# EUROPÄISCHE PATENTSCHRIFT

(11) **EP 1 874 382 B1**
(45) Veröffentlichungstag und Bekanntmachung des Hinweises auf die Patenterteilung: **30.05.2012**
(21) Anmeldenummer: 06707646.3
(22) Anmeldetag: 28.03.2006
(51) Int. Cl.: A61M 27/00, A61M 1/00

(54) **SCHLAUCHANSCHLUSS FÜR VAKUUMTHERAPIEGERÄT**
TUBE CONNECTOR FOR A VACUUM THERAPY DEVICE
RACCORD DE TUYAU POUR APPAREIL DE THERAPIE SOUS VIDE

(30) Priorität: 06.04.2005 DE 102005015878
(43) Veröffentlichungstag der Anmeldung: 09.01.2008
(73) Patentinhaber: VCS Medical Technology GmbH, 22525 Hamburg (DE)
(72) Erfinder: BAUMANN, Dirk, 20459 Hamburg (DE); MEYER, Johannes, 20259 Hamburg (DE); RÖHRS, Janine, 20257 Hamburg (DE)
(74) Vertreter: Kloiber, Thomas
(86) Internationale Anmeldenummer: PCT/EP2006/002794
(87) Internationale Veröffentlichungsnummer: WO 2006/105892

(56) Entgegenhaltungen:
- WO-A-02/089896
- FR-A- 2 876 592
- US-A- 4 392 854
- US-A- 5 014 389
- US-A- 5 352 211
- US-A- 5 437 651
- US-A1- 2002 161 346
- US-A1- 2003 109 855

## Beschreibung

Die vorliegende Erfindung betrifft ein therapeutisches Gerät zur Förderung der Heilung einer Wunde, umfassend eine Abdeckung um die Wunde herum zum Bilden einer im wesentlichen luftdichten Abdichtung über der Wunde, wobei zwischen Wunde und Abdeckung vorzugsweise ein für Flüssigkeiten durchlässiges Polster angeordnet ist, eine die Abdeckung mit einer Saugpumpe verbindende Drainageleitung, so dass an der Wunde gesaugt werden kann, um die Flüssigkeiten von dort abzuziehen, und einen Verbinder zum Verbinden der Abdeckung mit der Drainageleitung, wobei der Verbinder einen Durchgang zum Verbinden des proximalen Endes der Drainageleitung mit der Wunde aufweist.

Derartige Wundheilvorrichtungen dienen zur Behandlung sekundär verheilender chronischer oder akuter Wunden, insbesondere beim Menschen. Mit Hilfe des Unterdrucks um den die Wunde beinhaltenden Hautbereich soll Flüssigkeit, also insbesondere Wundsekret, von der Wunde wegtransportiert werden, um somit die Heilungsdauer zu verkürzen. In vielen Fällen wird durch den Einsatz einer derartigen Wundheilvorrichtung die Wundheilung überhaupt erst möglich gemacht.

Eine gattungsgemäße Wundheilvorrichtung ist aus der EP 1 088 569 B1 bekannt. Bei der in dieser Schrift offenbarten Wundheilvorrichtung ist insbesondere vorgesehen, dass der Verbinder eine scheibenartige Schale umfasst, deren untere Fläche mit dem porösen Polster in Kontakt steht. Dies soll offenbar einer gleichmäßigen Druckverteilung dienen. Der Verbinder der vorbekannten Wundheilvorrichtung ist vorgesehen für die Verwendung in Verbindung mit einem porösen Polster, welches auf die Wunde gedrückt wird. Das poröse Polster ist dehnbar. Eine gleichmäßige Verteilung des Drucks auf die Wunde wäre daher problematisch, da die Querschnittsfläche des Drainageschlauchs im allgemeinen deutlich kleiner sein wird als die Ausdehnung der Wunde. Es ist daher erforderlich, den vom Drainageschlauch eingetragenen Unterdruck mit oder ohne Verwendung eines durchlässigen Polsters auf eine möglichst große Fläche zu verteilen, damit die Wunde möglichst gleichmäßig über deren Ausdehnung mit dem Druck beaufschlagt ist. Hierzu wird in der zitierten Schrift vorgeschlagen, dass der Verbinder als scheibenartige Schale ausgebildet ist, wobei die untere Fläche dieser Schale mit dem porösen Polster in Kontakt steht.

In vielen Fällen der praktischen Anwendung ist jedoch diese bekannte Ausformung des Verbinders noch immer nicht ausreichend, um eine feste Verbindung zwischen der Abdeckung und dem Drainageschlauch sicherzustellen. Ferner ist die vorbekannte scheibenartige Schale nicht immer für die jeweilige Form der Wunde geeignet, da die vorgegebene Wölbung der Scheibe mitunter nicht passend sein kann. Die Form einer scheibenartigen Schale ist jedoch nur sehr begrenzt dehnbar. Zum Beispiel müßte ein Biegen einer konvex gekrümmten scheibenartigen Schale zur Wunde hin aus Gründen der Topologie zu einer Art Aufwulstung der Fläche führen. Außerdem ist die scheibenartige Form der Schale des Verbinders verhältnismäßig steif und kann sich insofern nicht an spezifische Wundenformen oder auch an Körperformen anpassen. Durch diese Nachteile bestehen Einschränkungen in der Verwendbarkeit der vorbekannten Wundheilvorrichtung.

Aus der US 2002/161346 A1 ist ebenfalls ein gattungsgemäßes therapeutisches Gerät bekannt, bei dem ein Verbinder zum Verbinden des Endes einer Drainageleitung mit der Wunde plattenförmig ausgestaltet ist. Auch dieser plattenförmige Verbinder ist relativ steif und kann sich daher nicht an Körperformen des Patienten bzw. Formen der Wunden anpassen. Es ist daher Aufgabe der vorliegenden Erfindung, ein gattungsgemäßes therapeutisches Gerät zur Förderung der Heilung einer Wunde anzugeben, mit welchem eine noch festere Verbindung zwischen der Abdeckung und der Drainageleitung sichergestellt wird, welche jedoch dennoch gleichzeitig eine ausreichende Elastizität zur Angleichung an die jeweilige Körper- und/oder Wundform aufweist.

Erfindungsgemäß wird diese Aufgabe dadurch gelöst, dass der Verbinder Befestigungsabschnitte aufweist, die sich von einem den Durchgang umschließenden Bereich ausgehend bis in den Bereich nahe des Außenrandes der Abdeckung erstrecken und durch Aussparungen voneinander getrennt sind, die als Unterbrechungen des äußeren Randes des Verbinders und in Richtung auf den Durchgang hin verjüngt ausgestaltet sind. Dadurch, dass die Befestigungsabschnitte sich bis nahe an den Außenrand der Abdeckung erstrecken, wird einerseits sichergestellt, dass der Druck ausgehend von der relativ kleinen Austrittsquerschnittsfläche des Drainageschlauches auf einen möglichst weiten Bereich der Wunde verteilt wird. Um jedoch bei einer derart großen Ausdehnung des Verbinders zu verhindern, dass dieser bei bestimmten ungünstigen Körper- und/oder Wundenformen nicht mehr gleichmäßig anliegt sondern vielmehr an einigen Stellen von der Wunde bzw. dem durchlässigen Polster beabstandet ist, sind andererseits zwischen einzelnen Befestigungsabschnitten Aussparungen vorgesehen. Diese Aussparungen zwischen einzelnen Befestigungsabschnitten stellen mit Vorteil eine gewisse Elastizität des Verbinders sicher, selbst wenn der Verbinder beispielsweise aus im wesentlichen steifem Material hergestellt ist. Der Vorteil liegt also in der Kombination einer gegenüber bekannten Verbindern vergrößerten Auflagefläche des Verbinders einerseits mit einer gleichzeitigen Sicherstellung einer gewissen Elastizität des verbreiterten Verbinders durch Aussparungen andererseits. Der äußere Rand des Verbinders befindet sich in dem größten radialen Abstand zur Achse des Durchgangs des Verbinders. Dies hat wie erläutert zur Folge, dass eine mögliche Abweichung der Krümmung des Verbinders von der Krümmung des durchlässigen Polsters dort maximal ist. Es ist aus diesem Grunde vorteilhaft, eben diese Randbereiche in Form von Aussparungen zu unterbrechen. Durch die Maßnahme, dass die Aussparungen in Richtung auf den Durchgang hin verjüngt ausgestaltet sind, wird der beschriebenen radialen Vergrößerung der Krümmungsdifferenz zwischen Verbinder und durchlässigem Polster in besonders effektiver Weise Rechnung getragen. Bekanntlich würde das Zusammendrücken des Außenrandes einer flexiblen scheibenartigen Schale gemäß dem Stand der Technik topologisch bedingt zu Aufwulstungen in Form von radial verlaufenden Falten führen. Dieses Problem wird gemäß der Erfindung durch die Verbreiterung der Aussparungen mit größerem Abstand von der Achse überraschend einfach gelöst.

Die Erfindung wird noch verbessert, wenn die Aussparungen bezüglich der Achse des Durchgangs im wesentlichen in radialer Richtung verlaufen. Insbesondere bei der typischen Situation, dass auf die Wunde ein durchlässiges Polster, wie etwa ein saugfähiger Schwamm, aufgelegt wird, ergibt sich eine mehr oder minder stark konvexe Fläche, auf die der Drainageschlauch mittels des Verbinders aufgesetzt werden muss. Die Krümmung dieser Fläche kann jedoch je nach dem Körperteil, an welchem sich die Wunde befindet, sowie je nach der Ausdehnung dieser Wunde variieren. Wenn also gemäß der Erfindung der Verbinder im Vergleich zu den bekannten Verbinder verbreitert werden soll, so dass er erfindungsgemäß bis nahe dem Rand der Abdeckung reicht, wird diese Variabilität der Krümmung der zu bedeckenden Fläche in zunehmendem Maße zu einem Problem, wie nachstehend erläutert. Die erfindungsgemäße Lösung dieses Problems durch die Aufteilung des Verbinders in Befestigungsabschnitte, welche jeweils durch Aussparungen voneinander getrennt sind, wird daher besonders wirksam, wenn gemäß dieser Ausgestaltung der Erfindung die Aussparungen radial zur Achse des Durchgangs verlaufen. Aus topologischen Gründen wird die Abweichung einer gekrümmten Fläche von einer ebenen Fläche mit zunehmendem radialen Abstand von einem Berührungspunkt dieser beiden Flächen immer größer. Wenn daher die Aussparungen ebenfalls in radialer Richtung zur Achse des Durchgangs verlaufen, kann diese Abweichung, welche sich in einer Beabstandung des gekrümmten Verbinders von dem andersartig gekrümmten durchlässigen Polster äußert, daher am wirksamsten vermieden werden. Durch in radialer Richtung verlaufende Aussparungen erreicht man, dass die Befestigungsabschnitte des erfindungsgemäßen Verbinders eine gewisse Elastizität erhalten, welche ohne die Aussparungen nicht gegeben wäre, da sich der erfindungsgemäße Verbinder im Unterschied zu bekannten Verbindern bis nahe an den Außenrand der Abdeckung erstreckt.

In spezieller Ausgestaltung der Erfindung ist der Verbinder im wesentlichen sternförmig ausgebildet. Die Symmetrie der Sternform führt mit Vorteil zu einer besonders gleichmäßigen Verteilung des Drucks über die Wunde.

In alternativer Ausgestaltung des erfindungsgemäßen therapeutischen Gerätes weisen die Aussparungen eine Spiralform auf. Hier kann eine beliebige Zahl von spiralartigen Aussparungen vom Außenrand des Verbinders in Richtung der Achse des Durchgangs vorgesehen sein. Die bereits genannten Probleme werden hierdurch ebenfalls gelöst.

Gemäß einer Variante der vorliegenden Erfindung bilden die Befestigungsabschnitte zur Wunde hin einen Hohlraum. Hierdurch wird durch den Verbinder nicht nur eine Verteilung des durch den Drainageschlauch eingeführten Drucks auf die Wunde erreicht, sondern zusätzlich noch eine Vergrößerung der Absaugefläche erzielt. Die
Absaugung von Wundsekret kann also zusätzlich durch die Hohlräume unter den Befestigungsabschnitten erfolgen. Ein unerwünschtes übermäßig langes Verbleiben von Wundsekret in der Wunde oder in einem darauf aufgelegten porösen Polster wird somit mit Vorteil wirksam vermieden.

Die Absaugung von Wundsekret über einen größeren Flächenbereich wird in Weiterbildung der Erfindung noch optimiert, wenn die Befestigungsabschnitte als in axialer Richtung aufgeschnittene Röhren ausgeformt sind, wobei vorzugsweise vom Durchmesser etwa 2/3 weggeschnitten sind. Der Transport von Wundsekret durch erfindungsgemäß nach unten geöffnete aufgeschnittene Röhren funktioniert besonders gut, da sich Röhren aufgrund ihrer kantenfreien Innenkonturen bekanntlich für den Transport von Flüssigkeiten hervorragend eignen. Damit der Hohlraum zwischen den aufgeschnittenen Röhren und der Wunde bzw. der porösen Wundauflage nicht übermäßig hoch wird und dabei dennoch einen ausreichend großen Querschnitt zum Absaugen aufweist, hat es sich als besonders günstig erwiesen, wenn vom Durchmesser der Röhren etwa 2/3 weggeschnitten sind. Selbstverständlich deutet die Formulierung "Wegschneiden" nicht notwendigerweise auf einen Herstellungsprozess für den erfindungsgemäßen Verbinder hin sondern bezieht sich vielmehr lediglich auf das dadurch erhaltene Ergebnis einer "Drittelröhre". Die Drittelröhre kann beispielsweise auch im Formgussverfahren hergestellt sein, ohne dass ein Wegschneiden erforderlich wäre.

Eine besonders symmetrische Druckverteilung ergibt sich in spezieller Ausgestaltung des erfindungsgemäßen therapeutischen Gerätes, wenn vier Befestigungsabschnitte vorgesehen sind.

Besonders kostengünstig und darüber hinaus auch sehr stabil ist eine Ausgestaltung der Erfindung, bei der der Verbinder aus einem zusammenhängenden Spritzgussteil hergestellt ist. Dabei können mit Vorteil sowohl die Befestigungsabschnitte als auch der Durchgang sowie ein möglicherweise vorgesehenes Anschlussstück für den Drainageschlauch sämtlich als zusammenhängendes Spritzgussteil hergestellt sein. Eine Montage der einzelnen Komponenten des Verbinders entfällt hierdurch, was vorteilhaft zur Kostenreduzierung führt. Ferner hat die Ausgestaltung als zusammenhängendes Spritzgussteil den Vorteil, dass praktisch nicht mit Undichtigkeiten des Verbinders zu rechnen ist.

Wenn in spezieller Ausgestaltung der Erfindung ein der Wunde abgewandtes Ende des Durchgangs mit einem abgewinkelten Anschlussstutzen versehen ist, werden Kräfte zwischen Schlauch und Verbinder, welche durch Bewegungsartefakte des Patienten entstehen können, wirksam aufgenommen. Insbesondere ermöglicht die Abwinklung des Anschlussstutzens eine Führung des Drainageschlauchs parallel zum Körper des Patienten, so dass eine Hebelwirkung an der Verbindungsstelle, die auftreten würde, wenn der Drainageschlauch senkrecht an den Verbinder angeschlossen wäre, vermieden wird. Insgesamt wird also mit Vorteil die Verbindung sicherer und robuster.

Eine Verteilung des Drucks über die Wunde wird in Ausgestaltung der Erfindung besonders wirksam erreicht, wenn der Verbinder aus im wesentlichen starrem Material hergestellt ist. Beispielsweise kann der Verbinder aus PVC oder anderen Kunststoffen hergestellt sein. Diese Materialien sind im Vergleich zu der Wundauflage sowie zu der Abdeckung und zu anderen Komponenten starr und können insofern als Druckverteiler dienen. Auf der anderen Seite weisen diese Materialien jedoch dennoch eine gewisse Elastizität auf. Die Elastizität ist besonders stark an den Extremitäten der Befestigungsabschnitte ausgeprägt, so dass in begrenztem Maße ein "Anschmiegen" der Befestigungsabschnitte des erfindungsgemäßen Verbinders an die Wunde oder das durchlässige Polster erreicht wird.

In Weiterbildung der Erfindung ist vorgesehen, dass an mindestens einem Befestigungsabschnitt ein Drucksensor angebracht ist. Da der Verbinder erfindungsgemäß im Unterdruckbereich der Wunde angebracht ist, eignet sich der Befestigungsabschnitt sehr gut als Druckmesspunkt. Beispielsweise kann dort ein Dehnungsmessstreifen zur Druckmessung angebracht sein. Der auf diese Weise gemessene Druck entspricht genau dem Druck vor Ort an der Wunde, der bei der Vakuumtherapie entscheidend ist. Dieser Messwert eignet sich daher bestens als Eingangsgröße für eine Druckregelung des Vakuumtherapiegerätes.

Besonders vorteilhaft ist es hier, wenn in Weiterbildung der Erfindung der Drucksensor in den Befestigungsabschnitt eingegossen ist. Hierdurch wird insbesondere mit Vorteil sichergestellt, dass sich der Drucksensor nicht von dem Verbinder lösen kann und in die Wunde eindringen kann. Außerdem ist durch das Eingießen des Drucksensors jeglicher Kontakt desselben mit der Wunde selber oder mit Wundsekret ausgeschlossen.

Für einen direkten Kontakt zwischen der Wunde oder der porösen Wundauflage ist es in Ausgestaltung der Erfindung von Vorteil, wenn der Verbinder unter der Abdeckung angeordnet ist. Alternativ ist es zwar auch denkbar, den Verbinder über der Abdeckung anzuordnen, jedoch ist dann einerseits kein direkter Kontakt zwischen Verbinder und Wunde bzw. porösem Polster mehr gegeben, andererseits können dann die Befestigungsabschnitte nicht mehr als Druckmesspunkte verwendet werden. Zudem ist eine Absaugung über die Befestigungsabschnitte ebenfalls nur möglich, wenn der Verbinder unter der Abdeckung angeordnet ist.

Gemäß einer Variante des erfindungsgemäßen therapeutischen Gerätes umfasst das Polster einen Naturschwamm, auf dem sich der Verbinder abstützt. Gegenüber der bereits bekannten Verwendung von Synthetikmaterialien wie zum Beispiel offenzelligen Polymerkunststoffen als poröses Polster für Vakuumtherapiegeräte weisen Naturschwämme eine Reihe von Vorteilen auf. Zunächst ist eine verstärkte Nachfrage seitens der Patienten nach natürlichen oder naturnahen Produkten zu beobachten. Darüber hinaus haben Naturschwämme in Form von Kompressen blutstillende Eigenschaften. Sie sind außerdem auch zur Wundreinigung geeignet. Darüber gibt es Hinweise auf biologisch aktive Inhaltsstoffe von Naturschwämmen, welche antibakteriell, entzündungshemmend, zytotoxisch oder mirostatisch wirken. Diese zahlreichen vorteilhaften Eigenschaften von Naturschwamm sind auch für die Vakuumwundheiltherapie von Vorteil. Darüber hinaus sind für den Einsatz in einem serienmäßigen kommerziellen Therapiegerät Naturschwämme in ausreichender Menge auf dem Markt in der gewünschten Qualität verfügbar. Die Kombination eines Naturschwamms mit dem erfindungsgemäßen Verbinder zur gleichmäßigen Verteilung des eingebrachten Drucks führt im Ergebnis zu einer optimalen Versorgung der Wunde.

Die Erfindung wird in einer bevorzugten Ausführungsform unter Bezugnahme auf eine Zeichnung beispielhaft beschrieben, wobei weitere vorteilhafte Einzelheiten den Figuren der Zeichnung zu entnehmen sind.

Funktionsmäßig gleiche Teile sind dabei mit denselben Bezugszeichen versehen.

Die Figuren der Zeichnung zeigen im Einzelnen:
- Fig. 1: schematische Darstellung der erfindungsgemäßen Wundheilvorrichtung sowie deren Funktionsweise
- Fig. 2: perspektivische Darstellung eines auf der Wunde aufgebrachten erfindungsgemäßen Verbinders zwischen Drainageschlauch und Wundabdeckung
- Fig. 3: Einzeldarstellung des erfindungsgemäßen Verbinders in Draufsicht
- Fig. 4a: seitliche Schnittdarstellung entlang der Linie A-A des Verbinders aus Fig. 3
- Fig. 4b: perspektivische Darstellung eines Verbinders aus der Figur 3 von unten
- Fig. 5: Prinzipdarstellungen der Form zweier Varianten des erfindungsgemäßen Verbinders

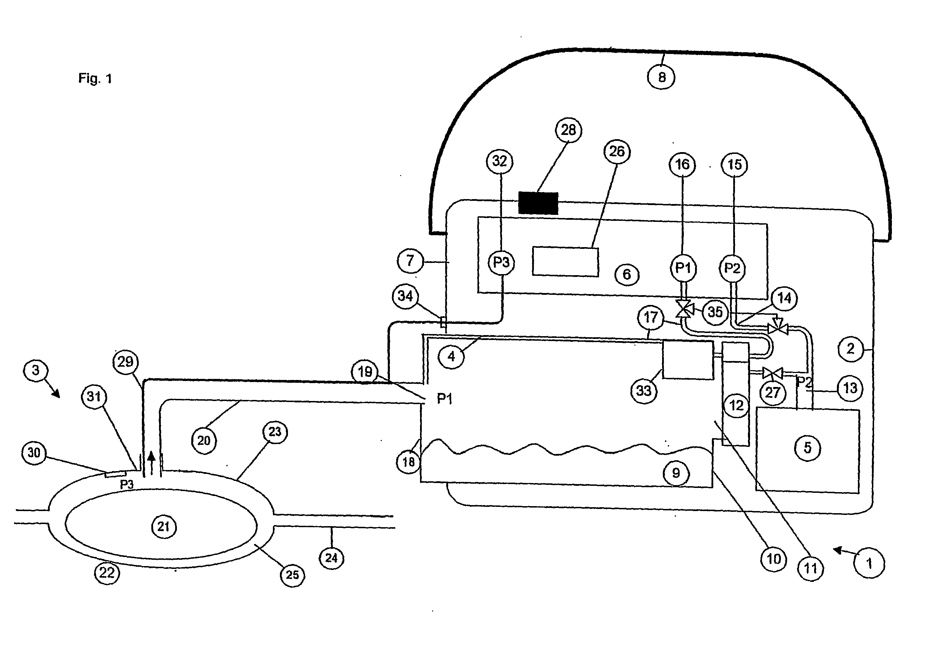

Die Figur 1 zeigt schematisch ein Ausführungsbeispiel einer erfindungsgemäßen Wundheilvorrichtung 1. Die Wundheilvorrichtung 1 besteht aus einer Basiseinheit 2 und einer Wundauflageeinheit 3. In der Basiseinheit 2 befindet sich ein wechselbarer Auffangbehälter 4, eine Saugpumpe 5 sowie ein Regel- und Steuergerät 6. Die Basiseinheit 2 ist von einem Gehäuse 7 umgeben. An dem Gehäuse 7 ist ein Tragegurt 8 angebracht.

In dem Auffangbehälter 4 befindet sich ein Gemisch 9 aus Wundsekret und Geliermittel. Der Auffangbehälter 4 weist an einer dem Gehäuse 7 zugewandten Innenwand 10 eine Absaugöffnung 11 auf. Die Absaugöffnung 11 ist über den Filter 12 über einen Absaugschlauch 13 mit der Saugpumpe 5 verbunden. Von dem Absaugschlauch 13 ist ein pumpenseitiger Sondenschlauch 14 abgezweigt. Der pumpenseitige Sondenschlauch 14 führt den im Absaugschlauch 13 herrschenden Druck p2 zu einem saugpumpenseitigen Drucksensor 15, welcher sich in dem Regel- und Steuergerät 6 befindet. In dem Regel- und Steuergerät 6 befindet sich ferner der Distaldrucksensor 16, welcher über den Sondenschlauch 17 sowie den Distalsondenschlauch 36 mit dem am distalen Ende des Absaugschlauches 13 herrschenden Druck p1 kommuniziert. Sowohl in dem Sondenschlauch 17 als auch in dem Sondenschlauch 14 ist jeweils ein 3/2-Wegeventil 35 eingebaut. Der Distalsondenschlauch 36 ist entlang des wechselbaren Auffangbehälters 4 innerhalb des Gehäuses 7 zu einer in einer Außenwand 18 des Auffangbehälters 4 befindlichen Ansaugöffnung 19 im Gehäuse 7 der Basiseinheit 2 geführt. Außerdem kann der Distalsondenschlauch 36 optional durch eine Blackbox 33 geführt sein.

Die Wundauflageeinheit 3 besteht aus dem Wundsekretabsaugschlauch 20, einem Schwamm 21 sowie einer Folie 23, durch welche der Wundsekretabsaugschlauch 20 und der Schwamm 21 so auf die die Wunde 22 umgebende Haut 24 aufgeklebt sind, dass eine luftdichte Verbindung entsteht. Der Schwamm 21 ist in der Lage, Flüssigkeiten aufzunehmen und zu speichern. Der Schwamm 21 wird durch die gespannte Folie 23 mit einem gewissen Anpressdruck in die Wunde 22 eingebracht. Zwischen Schwamm 21 und Folie 23 ist ein Verbinder 31 vorgesehen. An dem Verbinder 31 ist der Drainageschlauch 20 angeschlossen. Der Verbinder 31 berührt an dessen der Wunde 22 zugewandten Seite den Schwamm 21.

In der Wundauflage 3 ist ferner zwischen der Folie 23 und dem Schwamm 21 in der Nähe des Verbinders 31 ein Dehnungsmessstreifen 30 befestigt. Der Dehnungsmessstreifen 30 dient zur Messung des Wunddrucks an der Wunde 22. Der Dehnungsmessstreifen 30 ist mit einem elektrischen Kabel 29 verbunden. Das elektrische Kabel 29 ist mit dem äußeren Rand des Absaugschlauches 20 verbunden und auf diese Weise mittels einer Steckverbindung 34 im Gehäuse 7 der Basiseinheit 2 mit dem Auswertegerät 32 für den Wunddruck P3 verbunden. Das Auswertegerät 32 ist Bestandteil des Regel- und Steuergerätes 6.

Der Auffangbehälter 4, der Wundsekretabsaugschlauch 20, der Schwamm 21 und die Folie 23 sind jeweils steril und auswechselbar zum Einmalgebrauch. In der Wunde 22 befindet sich Wundsekret 25.

Zur Verwendung der erfindungsgemäßen Wundheilvorrichtung 1 wird der Schwamm 21 in die Wunde 22 eingebracht. Mit dem Schwamm 21 wird über den Verbinder 31 ein Ende des Wundsekretabsaugschlauchs 20 verbunden und anschließend werden der Wundsekretabsaugschlauch 20, der Verbinder 31 sowie der Schwamm 21 mit Hilfe der Folie 23 auf den die Wunde 22 umgebenden Hautbereich 24 derart aufgeklebt, dass dieses System luftdicht abgeschlossen ist.

Das andere Ende des Wundsekretabsaugschlauchs 20 wird mit der in der Außenwand 18 des Auffangbehälters 4 befindlichen Ansaugöffnung 19 ebenfalls luftdicht verbunden. Durch die Eigenschaft des Schwammes 21, Flüssigkeiten aufnehmen und speichern zu können, wird Wundsekret 25 aus der Wunde 22 in den Schwamm 21 abgeführt. Die die Speicherkapazität des Schwammes 21 übersteigende Wundsekretmenge wird nun über den Wundsekretabsaugschlauch 20 in den Auffangbehälter 4 abgesaugt. Der für die Absaugung erforderliche Unterdruck wird dabei von der Absaugpumpe 5 erzeugt.

Dazu kommuniziert der Absaugschlauch 13 der Saugpumpe 5 mit dem Auffangbehälter 4. Der zwischen dem Auffangbehälter 4 und dem Absaugschlauch 13 angeordnete Filter 12 verhindert dabei, dass Wundsekret 25 aus dem Auffangbehälter 4 in den Absaugschlauch 13, die Absaugpumpe 5, in den saugpumpenseitigen Sondenschlauch 14 oder gar in den saugpumpenseitigen Drucksensor 15 eintritt.

Das auf die beschriebene Weise in dem Auffangbehälter 4 gesammelte überschüssige Wundsekret 25 wird in dem Auffangbehälter 4 durch ein Geliermittel verdickt. Hierdurch wird ein Befeuchten des Filters 12 auch bei Lagewechsel der Basiseinheit 2 wirksam verhindert. Dadurch wird z.B. verhindert, dass der Filter 12 befeuchtet wird und ein Übertritt von Keimen in das Steuergerät 6 erfolgen könnte. Außerdem ist eine hygienischere Entsorgung der potentiell verunreinigten Wundflüssigkeit im festen Aggregatzustand möglich.

Der von der Saugpumpe 5 erzeugte Unterdruck wird von dem saugpumpenseitigen Drucksensor 15 einerseits sowie von dem wundseitigen Dehnungsmessstreifen 30 andererseits und außerdem über den Distaldruckaufnehmer 16, welcher den Distaldruck p1 misst, kontinuierlich aufgenommen. In dem Mikroprozessor des Regel- und Steuergerätes 6 wird anschließend eine Auswertung des durch den wundseitigen Dehnungsmessstreifen 30 aufgenommenen Drucks p3, des durch den saugpumpenseitigen Drucksensor 15 aufgenommenen Drucks p2 sowie des am distalen Ende des Absaugschlauchs 20 aufgenommenen Drucks p1 als Eingangsgrößen für die Regelung ausgewertet.

Zur Aufrechterhaltung eines feuchten Wundmilieus wird das Druckniveau in der Wundauflageeinheit 3 nach den individuellen Bedürfnissen des Patienten sowohl bezüglich der Eigenschaften der Wunde 22 als auch nach Kriterien des Verträglichkeitsempfindens (Schmerzen des Patienten) eingestellt.

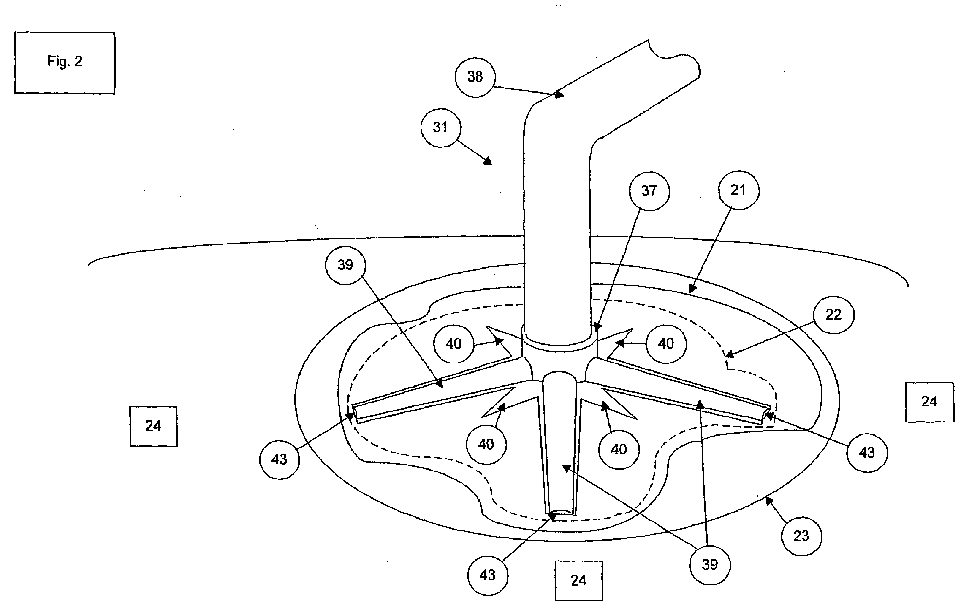

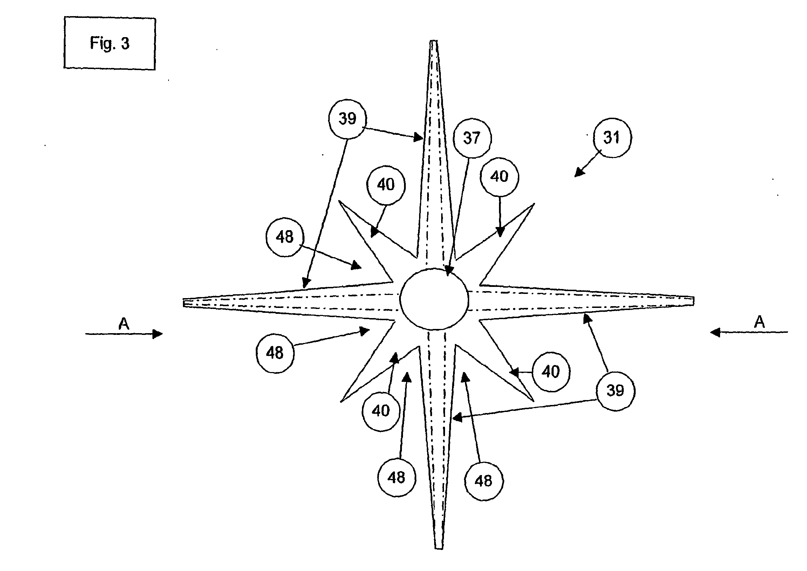

In der Figur 2 ist der Verbinder 31 im Detail zu erkennen. Der Verbinder 31 ist auf den Schwamm 21 aufgelegt. Der Schwamm 21 bedeckt seinerseits die Wunde 22 vollständig. Der Rand der Wunde 22 ist gestrichelt dargestellt, um anzudeuten, dass die Wunde 22 vollständig von dem Schwamm 21 bedeckt ist und somit in dieser Ansicht eigentlich nicht zu erkennen ist. Die durchsichtige Folie 23 ist über den Verbinder 31 gelegt und mit der die Wunde 22 umgebenden Haut 24 derart verklebt, dass eine im wesentlichen luftdichte Abdichtung entsteht. Der Verbinder 31 ist als zusammenhängendes Spritzgussteil ausgeführt. Als Material ist beispielsweise PVC (Polyvinylchlorid) geeignet. Es ist jedoch auch jeder andere Kunststoff oder sonstige Werkstoff geeignet, welcher zum Einsatz in medizinischen Therapiegeräten geeignet ist und im wesentlich starr ist mit einer gewissen Elastizität. Der Verbinder 31 weist am oberen Ende einen Durchgang 37 auf. Auf den Durchgang 37 ist ein Anschlussstutzen 38 aufgesetzt. Der Anschlussstutzen 38 ist abgewinkelt. Am Ende des Anschlussstutzens 38 befindet sich ein nicht näher dargestellter Anschluss für den Drainageschlauch 20. Der Durchgang 37 des Verbinders 31 mündet in vier Ansaugarme 39. In der Figur 2 ist einer der Ansaugarme 39 bedingt durch die Perspektive von dem Anschlussstutzen 38 verdeckt und daher nicht zu erkennen. Außerdem schließen sich wundseitig an den Durchgang 37 des Verbinders 31 vier Befestigungsarme 40 an. Die Ansaugarme 39 sind etwa dreimal so lang wie die Ansaugarme 39. Der doppelsternartige Grundriss des Verbinders 31 ist auch in der Draufsicht in Figur 3 zu erkennen.

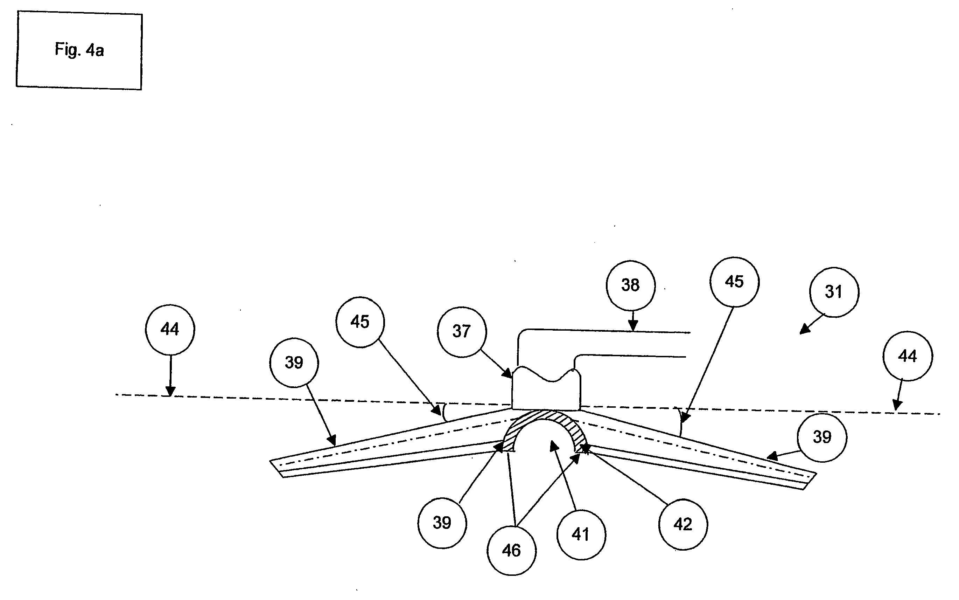

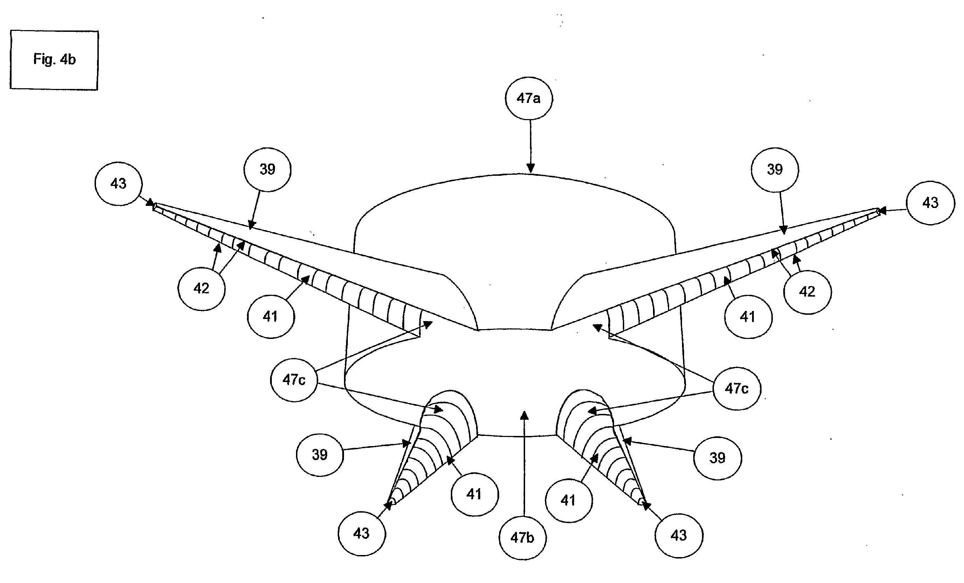

Die Ansaugarme 39 haben, wie in Figur 4 zu erkennen, im Querschnitt die Form einer in Längsrichtung aufgeschnittenen Röhre. Jeder Ansaugarm 39 umgrenzt somit mit seiner Wand 42 einen Kanal 41, welcher der Wunde 22 zugewandt ist. Sowohl die Befestigungsarme 40 als auch die Ansaugarme 39 verjüngen sich von dem Durchgang 37 nach außen hin. Die Befestigungsarme 40 verlaufen in dem Ausführungsbeispiel zum Ende hin spitz. Die Ansaugarme 39 erstrecken sich bis nahe an den Rand der Folie 23. Die Stirnflächen 43 der Ansaugarme 39 sind geschlossen. Dadurch ist der Kanal 41 nach oben durch die Wand 42 und zum Ende hin durch die Stirnfläche 43 begrenzt. Der Kanal 41 ist jedoch nach unten, d. h. zur Wunde 22 hin, geöffnet. In der Figur 3 ist im Interesse einer besseren Übersichtlichkeit der Anschlussstutzen 38 für den Drainageschlauch 20 nicht dargestellt. Die Befestigungsarme 40 sind im Unterschied zu den Absaugarmen 39 nicht konvex und bilden insofern auch keinen Kanal. Statt dessen bilden die Unterseiten der Befestigungsarme 40 eine plane Auflagefläche, welche auf den Schwamm 21 aufgelegt werden kann.

Wie in der Figur 4 zu erkennen, sind die Ansaugarme 39 jeweils um einen Winkel 45 zur Wunde abgewinkelt. Die Befestigungsarme 40 sind um den gleichen Winkel 45 zur Wunde geneigt. Die Unterflächen der Befestigungsarme 40 sowie die Endflächen 46 der Ansaugarme 39 liegen daher auf einer gedachten gemeinsamen Mantelfläche eines Kegels.

Beim Betrieb der Wundauflageeinheit 3 wird der von der Saugpumpe 5 auf den Drainageschlauch 20 übertragende Unterdruck mit Hilfe des erfindungsgemäßen Verbinders 31 auf die Wunde übertragen. Dazu wird die Saugwirkung am proximalen Ende des Drainageschlauchs 20 an dem Durchgang 37 des Verbinders 31 auf die Kanäle 41 der vier Ansaugarme 39 verteilt. Hierdurch wird der Verbinder 31 gleichmäßig durch den umgebenden Atmosphärendruck auf den Schwamm 21 gedrückt. Ein Verschieben des Verbinders 31 auf dem Schwamm 21 wird dabei vor allem über die Befestigungsarme 40 verhindert, da die Befestigungsarme 40 eine große Auflagefläche auf dem Schwamm 21 aufweisen. Der Verbinder 31 wirkt daher als Druckverteiler auf der Wunde. Der Druck wird dabei bis nahe an die Ränder der Folie 23 übertragen. Die Befestigungsarme 40 sowie insbesondere die Ansaugarme 39 sind dabei etwas elastisch, und können sich daher an eine Wölbung des Schwamms 21 anpassen. Hierdurch ist stets ein enges Anliegen des Verbinders 31 auf dem Schwamm 21 sichergestellt. Neben der druckverteilenden Wirkung wird auch die Effizienz der Absaugung des Wundsekrets 25, welches in dem Schwamm 21 gespeichert ist, deutlich verbessert. Die Verbesserung wird dadurch erreicht, dass über eine größere Fläche, nämlich die Öffnungsfläche der Kanäle 41 abgesaugt wird.

Die Verhältnisse bei der Absaugung sind besonders gut in der Figur 4 b zu erkennen. Dort ist ersichtlich, dass der Verbinder über insgesamt sechs Ausgänge 47 verfügt, und zwar der obere Ausgang 47a, an welchem der Drainageschlauch 20 angeschlossen ist, der dem Ausgang 47a gegenüberliegende, der Wunde zugewandte untere Ausgang 47b, sowie die vier in die Kanäle 41 der jeweiligen Absaugarme 39 mündenden Ausgänge 47c.

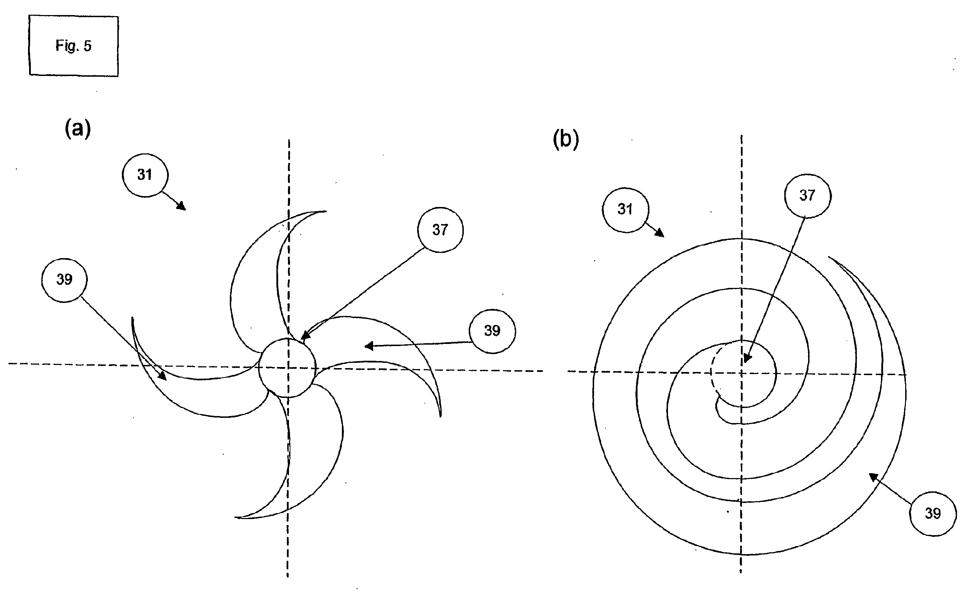

In der Figur 5 sind alternative Ausführungsformen des Verbinders 31 schematisch dargestellt. Die Darstellungen entsprechen dabei der Draufsicht aus Figur 3 der oben beschriebenen Ausführungsform. In Figur 5 a ist eine Ausführung des Verbinders 31 gemäß der Erfindung gezeigt, bei welcher die Ansaugarme 39 eine Spiralform aufweisen. In Teil b der Figur 5 ist eine andere alternative Ausführungsform des Verbinders gemäß der vorliegenden Erfindung zu erkennen, bei der lediglich ein einziger spiralförmiger Absaugarm 39 vorgesehen ist. Dieser windet sich mehrfach um den Durchgang 37. Obwohl die der Erfindung zugrunde liegende Aufgabe durch einen Verbinder der allgemeinen Form nach Figur 5 b ebenfalls gelöst wird, ist diese Variante aus praktischen Gründen nicht bevorzugt. Die bevorzugte Ausführungsform ist vielmehr die in den Figuren 2 bis 4 a / b dargestellte, sternförmige Bauweise.

### BEZUGSZEICHENLISTE

- 1: Wundheilvorrichtung
- 2: Basiseinheit
- 3: Wundauflageeinheit
- 4: Auffangbehälter
- 5: Saugpumpe
- 6: Regel- und Steuergerät
- 7: Gehäuse
- 8: Tragegurt
- 9: Gemisch
- 10: Innenwand
- 11: Absaugöffnung
- 12: Filter
- 13: Absaugschlauch
- 14: saugpumpenseitiger Sondenschlauch
- 15: saugpumpenseitiger Drucksensor
- 16: wundseitiger Drucksensor
- 17: wundseitiger Sondenschlauch
- 18: Außenwand
- 19: Ansaugöffnung
- 20: Wundsekretabsaugschlauch
- 20a: innere Schlauchwand
- 20b: äußere Schlauchwand
- 20c: Hohlraum
- 21: Schwamm
- 22: Wunde
- 23: Folie
- 24: Haut
- 25: Wundsekret
- 26: Anzeigeeinheit
- 27: Regelventil
- 28: Computerinterface
- 29: elektrisches Kabel
- 30: Dehnungsmessstreifen
- 31: Schlauchanschluss
- 32: P3 Auswertegerät
- 33: Blackbox
- 34: Steckverbindung
- 35: 3/2 Wegeventil
- 36: distaler Sondenschlauch
- 37: Durchgang
- 38: Anschlussstutzen
- 39: Ansaugarm
- 40: Befestigungsarm
- 41: Kanal
- 42: Wand
- 43: Stirnflächen
- 44: Horizontale
- 45: Winkel
- 46: Endfläche
- 47a: Ausgang
- 47b: Ausgang
- 47c: Ausgang
- 47d: Ausgang
- 48: Aussparungen

## Patentansprüche

1. Therapeutisches Gerät (1) zur Förderung der Heilung einer Wunde (22), umfassend eine Abdeckung (23) um die Wunde (22) herum zum Bilden einer im wesentlichen luftdichten Abdichtung über der Wunde (22), wobei zwischen Wunde (22) und Abdeckung (23) vorzugsweise ein für Flüssigkeiten durchlässiges Polster (21) angeordnet ist, eine die Abdeckung (23) mit einer Saugpumpe (5) verbindende Drainageleitung (20), so dass an der Wunde (22) gesaugt werden kann, um die Flüssigkeiten von dort abzuziehen, und einen Verbinder (31) zum Verbinden der Abdeckung (23) mit der Drainageleitung (20), wobei der Verbinder (31) einen Durchgang zum Verbinden des proximalen Endes der Drainageleitung (20) mit der Wunde(22)aufweist,**dadurch gekennzeichnet,dass** der Verbinder (31) Befestigungsabschnitte (39, 40) aufweist, die sich von einem den Durchgang (37) umschließenden Bereich ausgehend bis in den Bereich nahe des Außenrandes der Abdeckung (23) erstrecken und durch Aussparungen (48) voneinander getrennt sind, die als Unterbrechungen des äußeren Randes des Verbinders (31) und in Richtung auf den Durchgang (37) hin verjüngt ausgestaltet sind.

2. Therapeutisches Gerät nach Anspruch 1, **dadurch gekennzeichnet, dass** die Aussparungen (48) bezüglich der Achse des Durchgangs 37 im wesentlichen in radialer Richtung verlaufen.

3. Therapeutisches Gerät nach Anspruch 1 oder 2, **dadurch gekennzeichnet, dass** der Verbinder (31) im wesentlichen sternförmig ausgebildet ist.

4. Therapeutisches Gerät nach Anspruch 1, **dadurch gekennzeichnet, dass** die Aussparungen (48) eine Spiralform aufweisen.

5. Therapeutisches Gerät nach einem der vorhergehenden Ansprüche, **dadurch gekennzeichnet,dass** die Befestigungsabschnitte (39) zur Wunde hin einen Hohlraum (41) bilden.

6. Therapeutisches Gerät nach dem Anspruch 5, **dadurch gekennzeichnet, dass** die Befestigungsabschnitte (39) als in axialer Richtung aufgeschnittene Röhren ausgeformt sind, wobei vorzugsweise vom Durchmesser etwa zwei Drittel weggeschnitten sind.

7. Therapeutisches Gerät nach einem der vorhergehenden Ansprüche, **dadurch gekennzeichnet,dass** vier Befestigungsabschnitte (39, 40) vorgesehen sind.

8. Therapeutisches Gerät nach einem der vorhergehenden Ansprüche, **dadurch gekennzeichnet, dass** der Verbinder (31) aus einem zusammenhängenden Spritzgussteil hergestellt ist.

9. Therapeutisches Gerät nach einem der vorhergehenden Ansprüche, **dadurch gekennzeichnet,dass** ein der Wunde (22) abgewandtes Ende des Durchgangs (37) mit einem abgewinkelten Anschlussstutzen (38) versehen ist.

10. Therapeutisches Gerät nach einem der vorhergehenden Ansprüche, **dadurch gekennzeichnet, dass** der Verbinder (31) aus im wesentlichen starrem Material hergestellt ist.

11. Therapeutisches Gerät nach einem der vorhergehenden Ansprüche, **dadurch gekennzeichnet, dass** an mindestens einem Befestigungsabschnitt (39, 40) ein Drucksensor angebracht ist.

12. Therapeutisches Gerät nach Anspruch 11, **dadurch gekennzeichnet, dass** der Drucksensor in den Befestigungsabschnitt (39, 40) eingegossen ist.

13. Therapeutisches Gerät nach einem der vorhergehenden Ansprüche, **dadurch gekennzeichnet,dass** derVerbinder(31)unter der Abdeckung (23) angeordnet ist.

## Claims

1. Therapeutic device (1) to promote the healing of a wound (22), comprising a cover (23) around the wound (22) which forms a substantially airtight seal over the wound (22), a liquid-permeable pad (21) preferably being arranged between the wound (22) and the cover (23), a drainage line (20) connecting the cover (23) to a suction pump (5), in such a way that suction can be applied to the wound (22) in order to draw the fluids therefrom, and a connector (31) which connects the cover (23) to the drainage line (20), the connector (31) comprising an opening for connecting the proximal end of the drainage line (20) to the wound (22), **characterised in that** the connector (31) comprises fastening portions (39, 40) which extend from a region surrounding the opening (37) into the region close to the outer edge of the cover (23) and are separated from one other by notches (48), which are configured as interruptions of the outer edge of the connector (31) and taper in the direction of the opening (37).

2. Therapeutic device according to claim 1, **characterised in that** the notches (48) extend substantially in a radial direction with respect to the axis of the opening (37).

3. Therapeutic device according to either claim 1 or claim 2, **characterised in that** the connector (31) is substantially star-shaped.

4. Therapeutic device according to claim 1, **characterised in that** the notches (48) have a spiral shape.

5. Therapeutic device according to any one of the preceding claims, **characterised in that** the fastening portions (39) form a cavity (41) towards the wound.

6. Therapeutic device according to claim 5, **characterised in that** the fastening portions (39) are moulded as tubes cut open in an axial direction, approximately two-thirds preferably being cut away from the diameter.

7. Therapeutic device according to any one of the preceding claims, **characterised in that** four fastening portions (39, 40) are provided.

8. Therapeutic device according to any one of the preceding claims, **characterised in that** the connector (31) is produced from a continuous injection-moulded part.

9. Therapeutic device according to any one of the preceding claims, **characterised in that** an end of the opening (37) facing away from the wound (22) has a bent connecting sleeve (38).

10. Therapeutic device according to any one of the preceding claims, **characterised in that** the connector (31) is produced from substantially rigid material.

11. Therapeutic device according to any one of the preceding claims, **characterised in that** a pressure sensor is fitted on at least one fastening portion (39, 40).

12. Therapeutic device according to claim 11, **characterised in that** the pressure sensor is integrally cast into the fastening portion (39, 40).

13. Therapeutic device according to any one of the preceding claims, **characterised in that** the connector (31) is arranged under the cover (23).

## Revendications

1. Appareil thérapeutique (1) pour favoriser la cicatrisation d'une plaie (22), comprenant un élément de couverture (23) autour de la plaie (22) pour former une garniture substantiellement hermétique à l'air sur la plaie (22), dans lequel, entre la plaie (22) et l'élément de couverture (23) est disposé de préférence un tampon (21) perméable aux fluides, une conduite de drainage (20) se reliant à une pompe d'aspiration (5) de telle sorte qu'une aspiration puisse être réalisée sur la plaie (22) pour en retirer les fluides et un connecteur (31) pour relier l'élément de couverture (23) à la conduite de drainage (20), le connecteur (31) présentant un passage pour relier l'extrémité proximale de la conduite de drainage (20) à la plaie (22), **caractérisé en ce que** le connecteur (31) présente des segments de fixation (39, 40) qui s'étendent d'une zone entourant le passage (37) jusqu'à la zone à proximité du bord extérieur de l'élément de couverture (23) et qui sont séparés les uns des autres par des évidements (48) qui sont configurés comme des interruptions du bord extérieur du connecteur (31) et vont en s'amincissant dans la direction du passage (37).

2. Appareil thérapeutique selon la revendication 1, **caractérisé en ce que** les évidements (48) évoluent dans la direction substantiellement radiale par rapport à l'axe du passage 37.

3. Appareil thérapeutique selon la revendication 1 ou 2, **caractérisé en ce que** le connecteur (31) est conçu substantiellement en forme d'étoile.

4. Appareil thérapeutique selon la revendication 1, **caractérisé en ce que** les évidements (48) présentent une forme en spirale.

5. Appareil thérapeutique selon l'une des revendications précédentes, **caractérisé en ce que** les segments de fixation (39) forment un espace creux (41) vers la plaie.

6. Appareil thérapeutique selon la revendication 5, **caractérisé en ce que** les segments de fixation (39) sont formés en tant que tubes coupés dans la direction axiale, environ deux tiers du diamètre étant coupés de préférence.

7. Appareil thérapeutique selon l'une des revendications précédentes, **caractérisé en ce que** quatre segments de fixation (39, 40) sont prévus.

8. Appareil thérapeutique selon l'une des revendications précédentes, **caractérisé en ce que** le connecteur (31) est fabriqué à partir d'une pièce moulée par injection correspondante.

9. Appareil thérapeutique selon l'une des revendications précédentes, **caractérisé en ce qu'**une extrémité du passage (37) détourée de la plaie (22) est dotée d'un élément de raccordement (38) courbé.

10. Appareil thérapeutique selon l'une des revendications précédentes, **caractérisé en ce que** le connecteur (31) est fabriqué en un matériau substantiellement rigide.

11. Appareil thérapeutique selon l'une des revendications précédentes, **caractérisé en ce qu'**à au moins un segment de fixation (39, 40) est aménagé un capteur de pression.

12. Appareil thérapeutique selon la revendication 11, **caractérisé en ce que** le capteur de pression est coulé dans le segment de fixation (39, 40).

13. Appareil thérapeutique selon l'une des revendications précédentes, **caractérisé en ce que** le connecteur (31) est disposé sous le revêtement (23).
